(19) **Europäisches Patentamt**
**European Patent Office**
**Office européen des brevets**

(11) **EP 2 691 546 B1**

(12) **EUROPEAN PATENT SPECIFICATION**

(45) Date of publication and mention
of the grant of the patent:
**12.07.2017 Bulletin 2017/28**

(21) Application number: **12765217.0**

(22) Date of filing: **20.03.2012**

(51) Int Cl.:
***C12Q 1/68*** *(2006.01)* ***C07H 21/04*** *(2006.01)*

(86) International application number:
**PCT/US2012/029790**

(87) International publication number:
**WO 2012/134884 (04.10.2012 Gazette 2012/40)**

(54) **IDENTIFICATION OF A NUCLEIC ACID TEMPLATE IN A MULTIPLEX SEQUENCING REACTION**

IDENTIFIZIERUNG EINER NUKLEINSÄUREVORLAGE IN EINER
MULTIPLEX-SEQUENZIERUNGSREAKTION

IDENTIFICATION D'UNE MATRICE D'ACIDES NUCLÉIQUES DANS UNE RÉACTION DE
SÉQUENÇAGE MULTIPLEX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.03.2011 US 201113077169**

(43) Date of publication of application:
**05.02.2014 Bulletin 2014/06**

(73) Proprietor: **Good Start Genetics, Inc.
Cambridge, MA 02139 (US)**

(72) Inventors:
• **UMBARGER, Mark
Brookline, MA 02446 (US)**

• **PORRECA, Gregory
Cambridge, MA 02138 (US)**

(74) Representative: **Kirkham, Nicholas Andrew et al
Graham Watt & Co. LLP
St. Botolph's House
7-9 St. Botolph's Road
Sevenoaks, Kent TN13 3AJ (GB)**

(56) References cited:
**WO-A1-96/19586 WO-A1-98/14275
WO-A2-2004/083819 US-A1- 2008 269 068
US-A1- 2010 330 619**

## Description

Related Application

The present application claims the benefit of and priority to U.S. nonprovisional application serial number 13/077,169, filed March 31, 2011, the content of which is incorporated by reference herein in its entirety.

Field of the Invention

The invention generally relates to methods for maintaining the integrity and identification of a nucleic acid template in a multiplex sequencing reaction.

Background

Sequencing-by-synthesis involves template-dependent addition of nucleotides to a template/primer duplex. Nucleotide addition is mediated by a polymerase enzyme and added nucleotides may be labeled in order to facilitate their detection. Single molecule sequencing has been used to obtain high-throughput sequence information on individual DNA or RNA. The ability to multiplex samples, i.e., pool different patient samples, is important for decreasing costs and increasing the throughput of sequencing-by-synthesis platforms.

One issue that presents itself in a multiplex sequencing reaction is maintaining accurate identification of a sample throughout the sequencing process. Next generation sequencing typically involves the generation of an in vitro library; arraying of nucleic acid templates into physically distinct locations on a solid support (and optional clonal amplification of the templates into spatially localized clusters); and the sequencing-by-synthesis reaction itself. In a multiplex sequencing reaction, a unique barcode sequence is typically attached to template nucleic acids from each sample prior to pooling of templates from different samples so that sequencing data can be mapped back to a particular sample.

Errors can occur during generation of the library, barcode attachment, and arraying and amplification steps. Generally, the sample preparation phase and the barcoding phase frequently introduce errors into the subsequent sequencing reaction. For example, prior to attaching barcodes to each sample, there is significant potential for cross-contamination of template nucleic acids from different samples because library construction steps typically are conducted in parallel for multiple different samples. Additionally, the barcoding process makes the assumption that the barcode oligonucleotides attached to a given set of template nucleic acids from a sample are pure, i.e., not mixed with any other barcode oligonucleotides. However, cross-contamination of barcode oligonucleotides can readily occur during synthesis, purification, resuspension at the manufacturer, or during manipulation in the laboratory. Both scenarios lead to incorrectly associating a sequencing read with a particular sample. Accordingly, methods for reducing or eliminating errors in such procedures are needed.

Summary

The invention generally provides methods for validating the results of a sequencing reaction and for enabling the detection of errors introduced in sample preparation and barcode attachment. The invention utilizes identifier nucleic acids that are uniquely associated with a template nucleic acid along with unique barcode oligonucleotides associated with the identifier and the template, such that following a sequencing reaction, an association of an identifier nucleic acid with an unexpected barcode oligonucleotide reveals contamination in a sample. In this way, false positive and/or false negative results are avoided by requiring correct association between the identifier nucleic acid and the barcode oligonucleotide.

The invention is especially useful in multiplex next-generation sequencing applications in which errors can have a significant impact on results. The invention is based upon introducing an identifier nucleic acid to a solution including a template nucleic acid and incorporating an identical barcode oligonucleotide into both the template and the identifier. The barcodes can be of any appropriate length (e.g., from about 2 to about 50 nucleotides) and any number of barcode sequences can be used. The identifier nucleic acid and the barcode oligonucleotide are unique to the sample, so that valid sequence data are confirmed by the correct association of the identifier nucleic acid with the particular barcode. In contrast, an association of an identifier nucleic acid with an unexpected barcode oligonucleotide reveals contamination in the sample associated with this barcode and thus allows those templates to be excluded from analysis. Thus, methods of the invention make it possible to determine whether the integrity of a sample was maintained during a multiplex sequencing reaction and that sample integrity was maintained during sample preparation, and prevent the assigning of sequence data to the wrong sample.

After barcode oligonucleotides have been incorporated into the template and identifier, the template and the identifier are sequenced. Sequencing may be by any method known in the art. Sequencing-by-synthesis is a common technique used in next generation procedures and works well with the instant invention. However, other sequencing methods can be used, including sequence-by-ligation, sequencing-by-hybridization; gel-based techniques and others. In general, sequencing involves hybridizing a primer to both the template and the identifier to form a template/primer duplex and a identifier/primer duplex, contacting the duplexes with a polymerase in the presence of detectably-labeled nucleotides under conditions that permit the polymerase to add nucleotides to the primers in a template-dependent manner. Signal from the detectable label is then used as to identify the incorporated base and the steps are sequentially repeated in order to determine the linear order of nucleotides in the template and the identifier. Exemplary detectable labels include radiolabels, florescent labels, enzymatic labels, etc. In particular embodiments, the detectable label may be an optically detectable label, such as a fluorescent label. Exemplary fluorescent labels include cyanine, rhodamine, fluorescien, coumarin, BODIPY, alexa, or conjugated multi-dyes.

Numerous techniques are known for detecting sequence and for identifying barcodes and some are exemplified below. However, the exact means for detecting and compiling sequence data does not affect the function of the invention described herein.

Another aspect of the invention provides methods for quantifying a contamination rate in a sequencing reaction. Those methods involve determining a number of barcode reads in a sequencing reaction that are correctly correlated with an identifier nucleic acid, in which the identifier nucleic acid is associated with a template nucleic acid, and the barcode reads are generated by a sequencing apparatus, determining a number of barcode reads in the sequencing reaction that are incorrectly correlated with the identifier nucleic acid, and obtaining a ratio of the two numbers, thereby quantifying a contamination rate in the sequencing reaction.

Another aspect of the invention provides methods for identifying a contamination in a batch of barcode oligonucleotides. Those methods involve preparing a plurality of batches of barcoded identifier nucleic acids, in which each batch comprises a unique identifier nucleic acid attached to a unique barcode oligonucleotide, pooling the batches, sequencing the pooled batches, and identifying barcode oligonucleotides that are improperly paired with identifier nucleic acids.

Brief Description of the Drawings

Figure 1 is a drawing showing a typical prior art workflow employed to prepare multiple samples for a multiplexed next generation sequencing reaction.
Figure 2 is a drawing showing the potential for cross-contamination during a typical prior art workflow employed to prepare multiple samples for a multiplexed next generation sequencing reaction.
Figure 3 is a drawing showing how methods of the invention are able to identify cross-contamination in a multiplex sequencing reaction.
Figure 4 is a drawing showing methods for quantifying purity of batches of barcode oligonucleotides.

Detailed Description

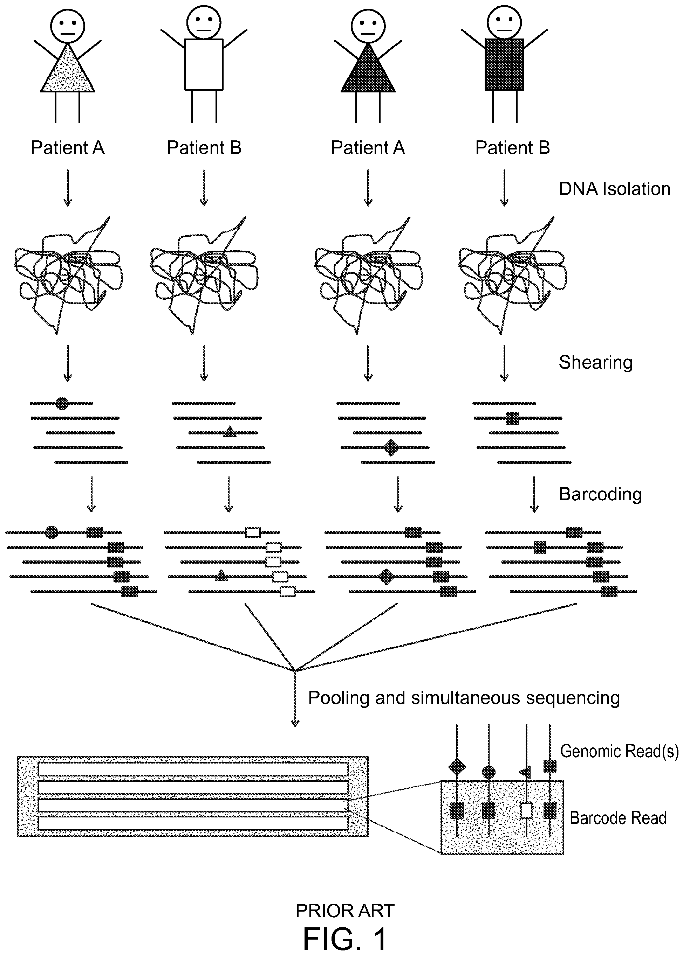

Reference is now made to Figure 1, which shows a typical prior art workflow employed to prepare multiple samples for a multiplexed next generation sequencing reaction. This workflow consists of two major steps, library construction and sequencing. During library construction, nucleic acid (e.g., DNA or RNA) is isolated from a sample and is subsequently fragmented into many smaller pieces. In some case, these smaller pieces may consist of random sections of the genome (e.g., a shotgun library), while in other cases the smaller pieces of nucleic acid may consist of specific regions of the genome that have been selected by a variety of possible approaches (e.g., molecular inversion probes, hybridization selection, Selector, or polymerase chain reaction). Next, barcoded oligonucleotides are attached to each smaller piece of nucleic acid by ligation or polymerase chain reaction. The barcode oligonucleotides are unique to nucleic acid from each sample such that no two samples have the same barcoded oligonucleotides. The barcodes serve to map from a given molecule to nucleic acid from a particular sample. Once barcoded, libraries are pooled, optionally amplified, and finally sequenced. The sequencing process consist of two reads, a read of the genomic region and a read of the barcode with the barcode read serving to allow the mapping of the genomic read to nucleic acid from a given sample. Alternatively, a single read which spans the barcode and genomic sequence could be performed and the resulting data could be split post-sequencing.

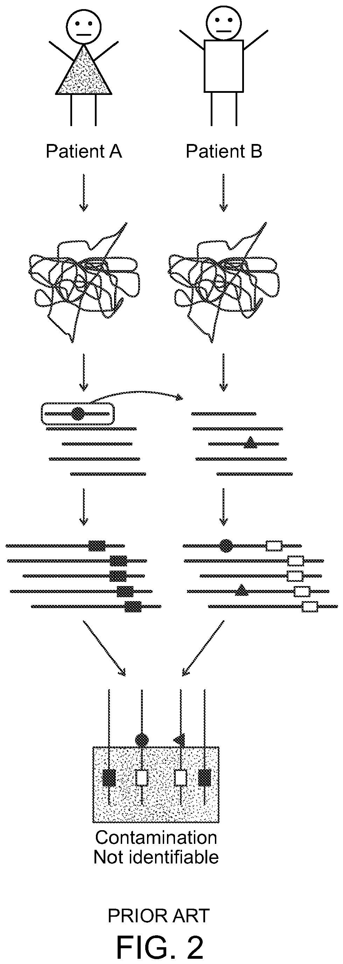

With the multiplexing workflow described in Figure 1, there are at least two general potential failures that could lead to the sequence of the genomic region of a given sample being associated with another sample. Reference is now made to Figure 2, which shows the potential for cross-contamination during a typical prior art workflow employed to prepare multiple samples for a multiplexed next generation sequencing reaction. A potential area for cross-contamination occurs during library preparation. Typically, library construction steps up to the point of barcoding are conducted in parallel for multiple different samples, involving multiple enzymatic steps and multiple purification steps. Hence, there is significant potential for cross-contamination of nucleic acids from one sample with those of another. This is shown in Figure 2 where a nucleic acid fragment containing a genomic region of interest from sample A becomes a contaminant in nucleic acid fragments from sample B during the library construction phase of the workflow. Since the barcode oligonucleotides are generally added at the last step in library construction, the nucleic acid from sample A will receive a barcode oligonucleotide that is associated with nucleic acids from sample B (Figure 2). Such cross-contamination cannot be identified on the sequencer and can lead to a molecule being mapped to the incorrect library. In this case, the nucleic acid from sample A will be associated with sample B (Figure 2).

Another source for sample cross-contamination are the batches of barcode oligonucleotides themselves. The barcoding process makes the assumption that the barcodes attached to a given patient's genomic regions are pure and are not mixed with any other barcode oligonucleotides. However, cross-contamination of barcodes can readily occur during synthesis, purification, resuspension at the manufacture, or during manipulation in a laboratory. As in the case with library cross-contamination, such barcode cross-contamination leads to an incorrect mapping of genomic reads to samples. Cross-contamination also occurs in the case of "molecular cross-over" during sample preparation and in the sequencing reaction itself.

Methods of the invention allow for identification of both library and barcode cross-contamination, and thus reduce or eliminate false positive and/or false negative results. Thus, methods of the invention make it possible to determine whether the integrity of a sample was maintained during a multiplex sequencing reaction, and prevent the assigning of sequence data to the wrong sample. Generally, methods of the invention involve utilizing specific identifier nucleic acids to track along with given barcoded samples. Following sequencing, an association of the sequence of an identifier nucleic acid with a barcode indicates cross-contamination of the sample and thus may provide reason for viewing data of a particular run with skepticism. In certain embodiments, methods of the invention involve obtaining a solution including a template nucleic acid, introducing an identifier nucleic acid to the solution, incorporating an identical barcode sequence into the template and the identifier nucleic acids, and sequencing the template and the identifier nucleic acids.

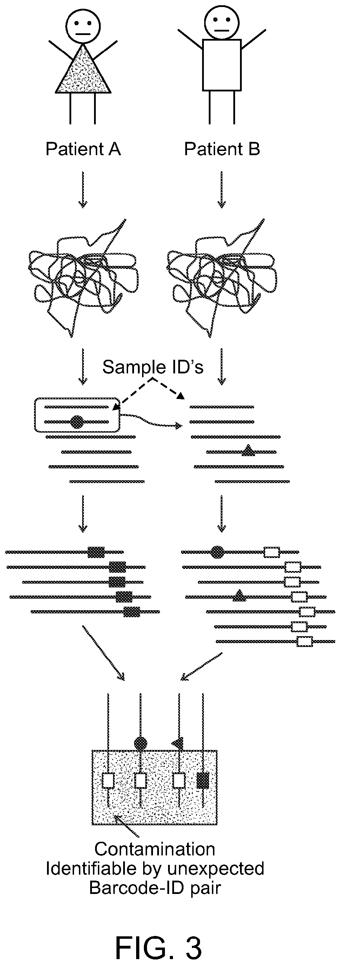

Reference is now made to Figure 3, which shows how methods of the invention are able to identify cross-contamination in a multiplex sequencing reaction. Methods of the invention involve introducing identifier nucleic acids into a solution including template nucleic acids from a sample. An identifier nucleic acid refers to a nucleic acid having a known sequence. The identifier may be either single-stranded or double-stranded. A unique identifier is added to each sample, and similar to a barcode oligonucleotide, the identifier nucleic acid can be used to track nucleic acids from different samples. The identifier nucleic acid may be added at any time during the library construction workflow prior to introduction of the barcode oligonucleotides. In certain embodiments, the identifier nucleic acid is introduced following nucleic acid purification.

As is shown in Figure 3, the identifier nucleic acids subsequently proceed through the library construction, barcoding, and sequencing workflow as if the identifier nucleic acids were molecules from a given sample. If there has been no contamination during the workflow, sequencing will demonstrate that each sample-specific identifier was paired with the expected barcode oligonucleotide. However, if there has been cross-contamination at any step during the workflow after the inclusion of the identifier nucleic acid, sample-specific identifier nucleic acids will have been paired with unexpected barcodes, and sequencing will produce data showing sample-specific identifiers paired with an incorrect barcode oligonucleotide, thereby flagging the data associated with that barcode(s) as erroneous data (Figure 3).

In certain embodiments, the identifier nucleic acids can be used to estimate the fraction of reads mapped to a particular library that are the result of cross-contamination. If $N_{correct \rightarrow A}$ and the number of bar code reads that map to an expected library identifier, and $N_{incorrect \rightarrow A}$ are the number of bar code reads that map to an unexpected library identifier, the rate of cross-contamination of library A with other libraries is estimated as:

$$\frac{N_{incorrect \rightarrow A}}{N_{correct \rightarrow A}} .$$

Using this fraction, one can define filters that correspond to desired levels of overall sequencing accuracy.

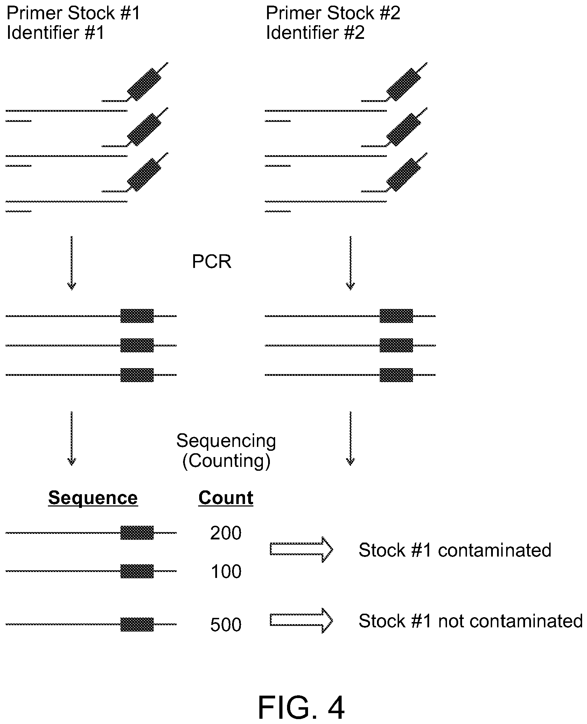

In certain embodiments, identifier nucleic acids can also be used to quantify the purity of barcode oligonucleotides, i.e., assess contamination upon batches of barcode oligonucleotides. Figure 4 shows a generally strategy for such methods. As described in further detail below, barcoded oligonucleotides can be attached to genomic regions to be sequenced by either PCR or ligation. To assess the purity of a barcoded PCR primer pair (or barcoded ligation oligonucleotides), a barcode identifier nucleic acid is designed and synthesized that contains a unique (and known) nucleic acid sequence flanked by a universal sequence of the barcode primers (ligation oligos) whose purity is to be assessed, PCR (ligation) is then performed using a single identifier as the template for amplification (ligation) with each barcode primer pair (ligation oligo). The products from the multiple barcode primer / identifier PCR (ligation) reactions are subsequently polled and the frequency at which a barcode identifier is associated with an (un)expected barcode is assessed by sequencing. A significant number of reads containing an unexpected pair is indicative of barcode stock contamination and can be utilized to identify primers that need to be resynthesized.

The following sections discuss general considerations for identifier nucleic acids, barcode oligonucleotides, attaching barcode oligonucleotides to nucleic acid templates and identifier nucleic acids, and nucleic acid sequencing, for example, template considerations, polymerases useful in sequencing-by-synthesis, choice of surfaces, reaction conditions, signal detection and analysis.

Nucleic Acid Templates

Nucleic acid templates include deoxyribonucleic acid (DNA) and/or ribonucleic acid (RNA). Nucleic acid templates can be synthetic or derived from naturally occurring sources, or may include both synthetic and natural sequence; and may include PCR products. In one embodiment, nucleic acid template molecules are isolated from a biological sample containing a variety of other components, such as proteins, lipids and non-template nucleic acids. Nucleic acid template molecules can be obtained from any cellular material, obtained from an animal, plant, bacterium, fungus, or any other cellular organism. Biological samples for use in the present invention include viral particles or preparations. Nucleic acid template molecules can be obtained directly from an organism or from a biological sample obtained from an organism, e.g., from blood, urine, cerebrospinal fluid, seminal fluid, saliva, sputum, stool and tissue. Any tissue or body fluid specimen may be used as a source for nucleic acid for use in the invention. Nucleic acid template molecules can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA.

Nucleic acid obtained from biological samples typically is fragmented to produce suitable fragments for analysis. In one embodiment, nucleic acid from a biological sample is fragmented by sonication. Nucleic acid template molecules can be obtained as described in U.S. Patent Application Publication Number US2002/0190663 A1, published Oct. 9, 2003. Generally, nucleic acid can be extracted from a biological sample by a variety of techniques such as those described by Maniatis, et al., Molecular Cloning: A Laboratory Manual, Cold Spring Harbor, N.Y., pp. 280-281 (1982). Generally, individual nucleic acid template molecules can be from about 1 base to about 20 kb. Nucleic acid molecules may be single-stranded, double-stranded, or double-stranded with single-stranded regions (for example, stem- and loop-structures).

A biological sample as described herein may be homogenized or fractionated in the presence of a detergent or surfactant. The concentration of the detergent in the buffer may be about 0.05% to about 10.0%. The concentration of the detergent can be up to an amount where the detergent remains soluble in the solution. In a preferred embodiment, the concentration of the detergent is between 0.1% to about 2%. The detergent, particularly a mild one that is nondenaturing, can act to solubilize the sample. Detergents may be ionic or nonionic. Examples of nonionic detergents include triton, such as the Triton® X series (Triton® X-100 t-Oct-$C_6H_4$-$(OCH_2$-$CH_2)_x$OH, x=9-10, Triton® X-100R, Triton® X-114 x=7-8), octyl glucoside, polyoxyethylene(9)dodecyl ether, digitonin, IGEPAL® CA630 octylphenyl polyethylene glycol, n-octyl-beta-D-glucopyranoside (betaOG), n-dodecyl-beta, Tween® 20 polyethylene glycol sorbitan monolaurate, Tween® 80 polyethylene glycol sorbitan monooleate, polidocanol, n-dodecyl beta-D-maltoside (DDM), NP-40 nonylphenyl polyethylene glycol, C12E8 (octaethylene glycol n-dodecyl monoether), hexaethyleneglycol mono-n-tetradecyl ether (C14EO6), octyl-beta-thioglucopyranoside (octyl thioglucoside, OTG), Emulgen, and polyoxyethylene 10 lauryl ether (C12E10). Examples of ionic detergents (anionic or cationic) include deoxycholate, sodium dodecyl sulfate (SDS), N-lauroylsarcosine, and cetyltrimethylammonium bromide (CTAB). A zwitterionic reagent may also be used in the purification schemes of the present invention, such as Chaps, zwitterion 3-14, and 3-[(3-cholamidopropyl)dimethylammonio]-1-propanesulf-onate. It is contemplated also that urea may be added with or without another detergent or surfactant.

Lysis or homogenization solutions may further contain other agents, such as reducing agents. Examples of such reducing agents include dithiothreitol (DTT), .beta.-mercaptoethanol, DTE, GSH, cysteine, cysteamine, tricarboxyethyl phosphine (TCEP), or salts of sulfurous acid.

Identifier nucleic acids

An identifier nucleic acid refers to a nucleic acid having a known sequence. The identifier nucleic acid may be single-stranded, double-stranded, or double-stranded with single-stranded regions (for example, stem- and loop-structures). Identifier nucleic acids can be natural, synthetic or may include both synthetic and natural sequence; and may include PCR product. The identifier nucleic acids can be designed so that they do not overlap the target region to be sequenced or contain a sequence that is identical to the target or the barcode oligonucleotides.

The identifier nucleic acids are designed such that the identifiers are correlated to a particular sample, allowing samples to be distinguished and validated, WO2004/083819 A2, WO96/19586. Methods of designing non-overlapping or unique sets of nucleic acids are shown for example in Brenner et al. (U.S. patent number 6,235,475.

Barcode Oligonucleotides

Methods of the invention involve attaching barcode oligonucleotides to or incorporated into a nucleic acid template and an identifier nucleic acid. Barcode oligonucleotides may be incorporated into a contiguous region of a template that includes the target to be sequenced. Exemplary methods for designing sets of barcode oligonucleotides and other methods for attaching barcode sequences are shown in U.S. paten numbers 6,138,077; 6,352,828; 5,636,400; 6,172,214; 6235,475; 7,393,665; 7,544,473; 5,846,719; 5,695,934; 5,604,097; 6,150,516; RE39,793; 7,537,897; 6172,218; and 5,863 722.

The barcode oligonucleotide generally includes certain features that make the oligonucleotide useful in sequencing reactions. For example the barcode oligonucleotides can be designed to have minimal or no homopolymer regions, i.e., 2 or more of the same base in a row such as AA or CCC, within the barcode sequence. The barcode oligonucleotides can also be designed so that they do not overlap the target region to be sequenced or contain a sequence that is identical to the target.

The barcode oligonucleotides are designed such that the sequences are correlated to a particular sample, allowing samples to be distinguished and validated. Methods of designing sets of barcode oligonucleotides is shown for example in Brenner et al. (U.S. patent number 6,235 475). In certain embodiments, the barcode oligonucleotides range from about 2 nucleotides to about 50; and preferably from about 4 to about 20 nucleotides. Since the barcode oligonucleotide is sequenced along with the template nucleic acid and the identifier nucleic acid, the oligonucleotide length should be of minimal length so as to permit the longest read from the template nucleic acid attached. Generally, the barcode oligonucleotides are spaced from the template nucleic acid molecule by at least one base.

Methods of the invention involve attaching the barcode oligonucleotides to the template nucleic acids and the identifier nucleic acids. Template nucleic acids are able to be fragmented or sheared to desired length, e.g. generally from 100 to 500 bases or longer, using a variety of mechanical, chemical and/or enzymatic methods. DNA may be randomly sheared via sonication, exposed to a DNase or one or more restriction enzymes, a transposase, or nicking enzyme. RNA may be fragmented by brief exposure to an RNase, heat plus magnesium, or by shearing. The RNA may be converted to cDNA before or after fragmentation.

Barcode oligonucleotides are integrated with template and identifier using methods known in the art. Barcode oligonucleotides are integrated with template and identifier using, for example, a ligase, a polymerase, Topo cloning (e.g., Invitrogen's topoisomerase vector cloning system using a topoisomerase enzyme), or chemical ligation or conjugation. The ligase may be any enzyme capable of ligating an oligonucleotide (RNA or DNA) to the template nucleic acid molecule. Suitable ligases include T4 DNA ligase and T4 RNA ligase (such ligases are available commercially, from New England Biolabs). Methods for using ligases are well known in the art. The polymerase may be any enzyme capable of adding nucleotides to the 3' and the 5' terminus of template nucleic acid molecules. Barcode oligonucleotides can be incorporated via a PCR reaction as part of the PCR primer.

The ligation may be blunt ended or via use of over hanging ends. In certain embodiments, following fragmentation, the ends of the fragments may be repaired, trimmed (e.g. using an exonuclease), or filled (e.g., using a polymerase and dNTPs), to form blunt ends. Upon generating blunt ends, the ends may be treated with a polymerase and dATP to form a template independent addition to the 3'-end and the 5-end of the fragments, thus producing a single A overhanging. This single A is used to guide ligation of fragments with a single T overhanging from the 5'-end in a method referred to as T-A cloning.

Alternatively, because the possible combination of overhangs left by the restriction enzymes are known after a restriction digestion, the ends may be left as is, i.e., ragged ends. In certain embodiments double stranded oligonucleotides with complementary over hanging ends are used.

Surface Attachment

Methods of the invention may involve attaching or immobilizing barcoded nucleic acid templates and barcoded identifier nucleic acids to solid supports. Such methods are described for example in Sabot et al. (U.S. patent application number 2009/0226975), Adessi et al. (U.S. patent number 7,115,400), and Kawashima et al. (U.S. patent application number 2005/01 00900). The term immobilized as used herein is intended to encompass direct or indirect attachment to a solid support via covalent or non-covalent bond(s). In certain embodiments of the invention, covalent attachment may be used, but generally all that is required is that template and identifier remain immobilized on the support. Typically, oligonucleotides are immobilized such that a 3' end is available for enzymatic extension and at least a portion of the sequence is capable of hybridizing to a complementary sequence. Immobilization can occur via hybridization to a surface attached oligonucleotide. Alternatively, immobilization can occur by means other than base-pairing hybridization, such as the covalent attachment set forth above.

Substrates or supports for use in the invention include, but are not limited to, latex beads, dextran beads, polystyrene surfaces, polypropylene surfaces, polyacrylamide gel, gold surfaces, glass surfaces and silicon wafers. In certain embodiments, the solid support may include an inert substrate or matrix that has been functionalized, for example by the application of a layer or coating of an intermediate material including reactive groups that permit covalent attachment to molecules such as polynucleotides.

Amplification

In certain embodiments, methods of the invention involve amplifying barcoded nucleic acid templates and barcoded identifier nucleic acids prior to sequencing the templates. Such methods are described for example in Sabot et al. (U.S. patent application number 2009/0226975), Adessi et al. (U.S. patent number 7,115,400), and Kawashima et al. (U.S. patent application number 2005/0100900).

Primer oligonucleotides or amplification sequences are polynucleotide sequences that are capable of annealing specifically to a single stranded polynucleotide sequence to be amplified under conditions encountered in a primer annealing step of an amplification reaction. Generally, the terms nucleic acid, polynucleotide and oligonucleotide are used interchangeably herein. The different terms are not intended to denote any particular difference in size, sequence, or other property unless specifically indicated otherwise. For clarity of description the terms may be used to distinguish one species of molecule from another when describing a particular method or composition that includes several molecular species.

Primers may additionally include non-nucleotide chemical modifications, for example to facilitate covalent attachment of the primer to a solid support. Certain chemical modifications may themselves improve the function of the molecule as a primer or may provide some other useful functionality, such as providing a cleavage site that enables the primer (or an extended polynucleotide strand derived therefrom) to be cleaved from a solid support. Useful chemical modifications can also provide reversible modifications that prevent hybridization or extension of the primer until the modification is removed or reversed. Similarly, other molecules attached to a surface in accordance with the invention can include cleavable linker moieties and or reversible modifications that alter a particular chemical activity of function of the molecule.

A plurality of oligonucleotides used in the methods set forth herein can include species that function as capture oligonucleotides. The capture oligonucleotides may include a template specific portion, namely a sequence of nucleotides capable of annealing to a primer binding sequence in a single stranded polynucleotide molecule of interest such as one that is to be amplified. The primer binding sequences will generally be of known sequence and will therefore be complementary to a region of known sequence of the single stranded polynucleotide molecule. The capture oligonucleotides may include a capture sequence and an amplification sequence. For example, a capture oligonucleotide may be of greater length than amplification primers that are attached to the same substrate, in which case the 5' end of the capture sequences may comprise a region with the same sequence as one of the amplification primers. A portion of a template, such as the 3' end of the template, may be complementary to the 3' of the capture sequences. The 5' end of the template may contain a region that comprises a sequence identical to one of the amplification primers such that upon copying the template, the copy can hybridize to the immobilized amplification primer. Thus, an oligonucleotide species that is useful in the methods set forth herein can have a capture sequence, an amplification sequence or both. Conversely, an oligonucleotide species can lack a capture sequence, an amplification sequence or both. In this way the hybridization specificity of an oligonucleotide species can be tailored for a particular application of the methods.

The length of primer binding sequences need not be the same as those of known sequences of polynucleotide template molecules and may be shorter, being particularly 16-50 nucleotides, more particularly 16-40 nucleotides and yet more particularly 20-30 nucleotides in length. The desired length of the primer oligonucleotides will depend upon a number of factors. However, the primers are typically long (complex) enough so that the likelihood of annealing to sequences other than the primer binding sequence is very low. Accordingly, known sequences that flank a template sequence can include a primer binding portion and other portions such as a capture sequence, barcode sequence or combination thereof.

In certain embodiments of the invention, amplification primers for solid phase amplification are immobilized by covalent attachment to the solid support at or near the 5' end of the primer, such that a portion of the primer is free to anneal to its cognate template and the 3' hydroxyl group is free to function in primer extension.

The chosen attachment chemistry will typically depend on the nature of the solid support and any functionalization or derivatization applied to it. In the case of nucleic acid embodiments, the primer itself may include a moiety which may be a non-nucleotide chemical modification to facilitate attachment. For example, the primer may include a sulfur containing nucleophile such as a phosphorothioate or thiophosphate at the 5' end. In the case of solid supported polyacrylamide hydrogels, this nucleophile may bind to a bromoacetamide group present in the hydrogel. In one embodiment, the means of attaching primers to the solid support is via St phosphorothioate attachment to a hydrogel comprised of polymerized acrylamide and N-(5-bromoacetamidylpentyl) acrylamide (BRAPA).

A uniform, homogeneously distributed lawn of immobilized oligonucleotides may be formed by coupling (grafting) a solution of oligonucleotide species onto the solid support. The solution can contain a homogenous population of oligonucleotides but will typically contain a mixture of different oligonucleotide species. The mixture can include, for example, at least two, three or more different species of oligonucleotide. Each surface that is exposed to the solution therefore reacts with the solution to create a uniform density of immobilized sequences over the whole of the exposed solid support. As such, a portion of the surface having a mixture of different immobilized sequences can be surrounded by an area of the surface having a mixture of the same immobilized sequences. A suitable density of amplification oligonucleotides is at least 1 fmol/mm$^2$ (6 x 10$^{10}$ per cm$^2$), or more optimally at least 10 fmol/mm$^2$ (6 x 10$^{11}$ per cm$^2$). The density of the capture oligonucleotides can be controlled to give an optimum cluster density of 10$^6$ - 10$^9$ clusters per cm$^2$ and optimum cluster brightness. The ratio of capture oligonucleotide species to the amplification oligonucleotide species can be any desired value including, but not limited to at least 1:100, 1:1000 or 1:100000 depending on the desired cluster density and brightness. Similar densities or ratios of other molecular species can be used in embodiments where molecules other than nucleic acids are attached to a surface.

In a particular embodiment, for each cluster of template molecules, a complementary copy of a single stranded polynucleotide template molecule is attached to the solid support by hybridization. Methods of hybridization for formation of stable duplexes between complementary sequences by way of Watson-Crick base-pairing are known in the art. The immobilized capture oligonucleotides can include a region of sequence that is complementary to a region or template specific portion of the single stranded template polynucleotide molecule. An extension reaction may then be carried out in which the capture sequence is extended by sequential addition of nucleotides to generate a complementary copy of the single stranded polynucleotide sequence attached to the solid support via the capture oligonucleotide. The single stranded polynucleotide sequence not immobilized to the support may be separated from the complementary sequence under denaturing conditions and removed, for example by washing.

The terms separate and separating, when used in reference to strands of a nucleic acid, refer to the physical dissociation of the DNA bases that interact within for example, a Watson-Crick DNA-duplex of the single stranded polynucleotide sequence and its complement. The terms also refer to the physical separation of these strands. Thus, the term can refer to the process of creating a situation wherein annealing of another primer oligonucleotide or polynucleotide sequence to one of the strands of a duplex becomes possible. After the first extension reaction, the duplex is immobilized through a single 5' attachment, and hence strand separation can result in loss of one of the strands from the surface. In cases where both strands of the duplex are immobilized, separation of the strands means that the duplex is converted into two immobilized single strands.

In one aspect of the invention, one or more of the amplification primers can be modified to prevent hybridization of a region or template specific portion of the single stranded polynucleotide molecule. Alternatively or additionally, one or more of the amplification primers may be modified to prevent extension of the primer during one or more extension reactions, thus preventing copying of the hybridized templates. These modifications can be temporary or permanent.

Generally, the capture sequences will include a region of the same sequence as the plurality of amplification oligonucleotides. Once the 3' end of the extended immobilized template copy has hybridized to one of the amplification primers and been extended, the resulting duplex will be immobilized at both ends and all of the bases in the capture oligonucleotide sequence will have been copied. Thus the capture oligonucleotide may include both the amplification primer sequence, plus a further sequence that is complementary to the end of the template. Typically the sequence complementary to the end of the template will not be present in any of the amplification primers. Alternatively, the amplification primers can contain the sequences complementary to the ends of the single stranded templates, but the amplification primers can be reversibly blocked to prevent hybridization and/or extension during one or more extension step, such as a first extension step in a particular amplification process.

According to one aspect of the invention, one or more of the amplification primers may include a modification that acts as a reversible block to either template hybridization or extension or both. By way of non-limiting example, such modifications can be presence of an additional sequence of nucleotides that is complementary to the amplification primer. This additional sequence can be present in a portion of the amplification primer and thus acts as an intramolecular hairpin duplex, or a 3' blocking group that prevents extension of the primer. Alternatively, the additional sequence can be found on a separate oligonucleotide that hybridizes to the amplification primer. A particular feature of such a modification is that it can be removed, altered or reversed such that the functionality of the modified primer oligonucleotide is restored and the primer is able to undergo hybridization and extension during later steps of the methods. Among other examples, the blocking group may be a small chemical species such as a 3' phosphate moiety that can be removed enzymatically, may be an a basic nucleotide such that the 3' end of the primer is not capable of hybridization (and thereby extension), or may be a sequence of nucleotides that can be selectively excised from the immobilized strands, for example, using restriction endonucleases that selectively cleave particular sequences or deglycosylases that selectively cleave oligonucleotides having exogenous bases such as uracil deoxyribonucleotides or 8-oxoguanine.

In one embodiment a plurality of three types of oligonucleotides (for example comprising capture sequences, forward and reverse primers) are immobilized to a solid support. Alternatively the three oligonucleotides may be forward amplification, blocked forward amplification and reverse amplification, where the unblocked forward primer acts as the capture sequence.

The single stranded polynucleotide molecules may have originated in single-stranded form, as DNA or RNA or may have originated in double-stranded DNA (dsDNA) form (e.g. genomic DNA fragments, PCR and amplification products and the like). Thus a single stranded polynucleotide may be the sense or antisense strand of a polynucleotide duplex. Methods of preparation of single stranded polynucleotide molecules suitable for use in the method of the invention using standard techniques are well known in the art. The precise sequence of the primary polynucleotide molecules may be known or unknown during different steps of the methods set forth herein. It will be understood that a double stranded polynucleotide molecule can be hybridized to an immobilized capture oligonucleotide as exemplified herein for single stranded polynucleotide molecules, so long as a single stranded region of the double stranded polynucleotide is available and at least partially complementary to the capture oligonucleotide sequence.

An exemplary method for the isolation of one strand of a double stranded molecular construct is described herein. A sample of unknown sequence may be fragmented and have barcode sequences attached at both ends of the fragment. Adapters are then attached to the ends of each fragment. One strand of the adapters may contain a moiety for surface immobilization, for example a biotin that can be captured onto a streptavidin surface. The adapters may be mismatch adapters, for example as described in co-pending application US 2007/0128624, Amplification of the mismatch or forked adapters using a pair of amplification primers, one of which carries a biotin modification means that one strand of each duplex carries a biotin modification. Immobilization of the strands onto a streptavidin surface means that the non-biotinylated strand can be eluted simply by denaturation/strand separation. The eluted constructs will be in single stranded form and upon exposure to hybridization conditions can be used to hybridize against the immobilized capture sequences which can be extended.

In a particular embodiment, the single stranded polynucleotide molecules are DNA molecules. More particularly, the single stranded polynucleotide molecules represent genomic DNA molecules, or amplicons thereof, which include both intron and exon sequence (coding sequence), as well as non-coding regulatory sequences such as promoter and enhancer sequences. Still yet more particularly, the single stranded polynucleotide molecules are human genomic DNA molecules, or amplicons thereof.

Methods of hybridization for formation of stable duplexes between complementary sequences by way of Watson-Crick base pairing are known in the art. A region or part of the single stranded polynucleotide template molecules can be complementary to at least a part of the immobilized capture sequence oligonucleotides. Since the amplification oligonucleotides are either modified to prevent hybridization and/or extension, or are non-complementary to the known ends of the template strands, only the capture sequences will be capable of hybridization and extension. An extension reaction may then be carried out wherein the capture sequence primer is extended by sequential addition of nucleotides to generate a complementary copy of the single stranded template polynucleotide attached to the solid support via the capture sequence oligonucleotide. The single stranded template polynucleotide sequence not immobilized to the support may be separated from the complementary sequence under denaturing conditions and removed, for example by washing. The distance between the individual capture sequence oligonucleotides on the surface therefore controls the density of the single stranded template polynucleotides and hence the density of clusters formed later on the surface is also controlled.

In certain embodiments in which the modified forward primer oligonucleotides are blocked and are unable to be extended, generally all of the amplification primer oligonucleotides will hybridize to the single stranded template polynucleotides. When the extension reaction is carried out only the unmodified forward capture primer oligonucleotides are extended by sequential addition of nucleotides to generate a complementary copy of the single stranded template polynucleotide attached to the solid support via the unmodified forward primer oligonucleotide. The single stranded template polynucleotide sequences not hybridized to the support may be separated from the un-extended blocked forward primer oligonucleotides under denaturing conditions and removed, for example by washing with a chemical denaturant such as formamide. The distance between the individual unmodified forward primer oligonucleotides on the surface therefore controls the density of the single stranded template polynucleotides and hence the density of clusters formed later on the surface is also controlled.

Following the attachment of the complementary single stranded template polynucleotides, the modified/blocked primers can be treated to reverse, remove or alter the modification such that they become functionally equivalent to the unmodified forward primer oligonucleotides. For example, the double stranded structure may be removed either by denaturation, for example by heating or treatment with an alkaline solution when it is formed by a separate hybridized polynucleotide. Alternatively, where the hybridized polynucleotide is covalently linked, enzymatic digestion could be used to sequence-selectively cleave the strand, followed by denaturation. Such methods for removing the double stranded structure are known in the art and would be apparent to the skilled person (Sambrook and Russell, Molecular Cloning, A Laboratory Manual, third edition, Cold Spring Harbor Laboratory Press (2001)).

In one embodiment of the invention, the single stranded template polynucleotide molecule can be attached to the solid support by ligation to double stranded primers immobilized to the solid support using ligation methods known in the art (Sambrook and Russell, supra). Such methods utilize ligase enzymes such as DNA ligase to effect or catalyze the joining of the ends of the two polynucleotide strands, in this case, the single stranded template polynucleotide molecule and the primer oligonucleotide ligate such that covalent linkages are formed. In this context "joining" means covalent linkage of two polynucleotide strands that were not previously covalently linked. Thus, an aim of the invention can also be achieved by modifying the 3' end of a subset of primer oligonucleotides such that they are unable to ligate to the single stranded template polynucleotides. By way of non-limiting example, the addition of 2'3'dideoxy AMP (dideoxyAMP) by the enzyme terminal deoxynucleotidyl transferase (TdT) effectively prevents T4 DNA ligase from ligating treated molecules together.

An alternative method would be to have the capture sequences as duplex strands and the amplification sequences as single strands. Upon ligation of the single strands to the capture duplexes (which would be the only immobilized species carrying a free 5' phosphate) the 3' end of the immobilized strand can be extended as described above. Upon denaturation of the hybridized template sequence, amplification of the immobilized strand can proceed as described. Other such methods for attaching single strands will be apparent to others skilled in the art.

In a next step according to particular embodiments of the present invention, suitable conditions are applied to the immobilized single stranded polynucleotide molecule and the plurality of amplification primer oligonucleotides such that the single stranded polynucleotide molecule hybridizes to an amplification primer oligonucleotide to form a complex in the form of a bridge structure. Suitable conditions such as neutralizing and/or hybridizing buffers are well known in the art (See Sambrook et al., supra; Ausubel et al., Current Protocols in Molecular Biology, John Wiley and Sons, Baltimore, Md. (1998)). The neutralising and/or hybridising buffer may then be removed.

Next by applying suitable conditions for extension an extension reaction is performed. The primer oligonucleotide of the complex is extended by sequential addition of nucleotides to generate an extension product complimentary to the single stranded polynucleotide molecule. The resulting duplex is immobilized at both 5' ends such that each strand is immobilized.

Suitable conditions such as extension buffers/solutions comprising an enzyme with polymerase activity are well known in the art (See Sambrook et al., supra; Ausubel et al. supra). In a particular embodiment dNTP's may be included in the extension buffer. In a further embodiment dNTP's could be added prior to the extension buffer. This bridge amplification technique can be carried out as described, for example, in Adessi et al. (U.S. patent number 7,115,400), and Kawashima et al. (U.S. patent application number 2005/0100900).

Examples of enzymes with polymerase activity which can be used in the present invention are DNA polymerase (Klenow fragment, T4 DNA polymerase), heat-stable DNA polymerases from a variety of thermostable bacteria (such as Taq, VENT, Pfu, or Tfl DNA polymerases) as well as their genetically modified derivatives (TaqGold, VENTexo, or Pfu exo). A combination of RNA polymerase and reverse transcriptase can also be used to generate the extension products. Particularly the enzyme has strand displacement activity, more particularly the enzyme will be active at a pH of about 7 to about 9, particularly pH 7.9 to pH 8+, yet more particularly the enzymes are Est or Klenow.

The nucleoside triphosphate molecules used are typically deoxyribonucleotide triphosphates, for example dATP, dTTP, dCTP, dGTP, or are ribonucleoside triphosphates for example ATP, UTP, CTP, GTP, The nucleoside triphosphate molecules may be naturally or non-naturally occurring.

After the hybridization and extension steps, the support and attached nucleic acids can be subjected to denaturation conditions. A flow cell can be used such that, the extension buffer is generally removed by the influx of the denaturing buffer. Suitable denaturing buffers are well known in the art (See Sambrook et al., supra; Ausubel et al. supra). By way of example it is known that alterations in pH and low ionic strength solutions can denature nucleic acids at substantially isothermal temperatures. Formamide and urea form new hydrogen bonds with the bases of nucleic acids disrupting hydrogen bonds that lead to Watson-Crick base pairing. In a particular embodiment the concentration of formamide is 50% or more. These result in single stranded nucleic acid molecules. If desired, the strands may be separated by treatment with a solution of very low salt (for example less than 0.01 M cationic conditions) and high pH (>12) or by using a chaotropic salt (e.g. guanidinium hydrochloride). In a particular embodiment a strong base is used. A strong base is a basic chemical compound that is able to deprotonate very weak acids in an acid base reaction. The strength of a base is indicated by its pK.sub.b value, compounds with a $pK_b$ value of less than about 1 are called strong bases and are well known to one skilled in the art. In a particular embodiment the strong base is Sodium Hydroxide (NaOH) solution used at a concentration of from 0.05 M to 0.25 M, particularly 0.1 M.

Following the hybridization, extension and denaturation steps exemplified above, two immobilized nucleic acids will be present, the first being the first template single stranded polynucleotide molecule (that was initially immobilized) and the second being a nucleic acid complementary thereto, extending from one of the immobilized primer oligonucleotides. Both the original immobilized single stranded polynucleotide molecule and the immobilized extended primer oligonucleotide formed are then able to initiate further rounds of amplification by subjecting the support to further cycles of hybridization, extension and denaturation.

It may be advantageous to perform optional washing steps in between each step of the amplification method. For example an extension buffer without polymerase enzyme with or without dNTP's could be applied to the solid support before being removed and replaced with the full extension buffer.

Such further rounds of amplification can be used to produce a nucleic acid colony or cluster comprising multiple immobilized copies of the single stranded polynucleotide sequence and its complementary sequence.

The initial immobilization of the single stranded polynucleotide molecule means that the single stranded polynucleotide molecule can hybridize with primer oligonucleotides located at a distance within the total length of the single stranded polynucleotide molecule. Other surface bound primers that are out of reach will not hybridize to the polynucleotide. Thus the boundary of the nucleic acid colony or cluster formed is limited to a relatively local area surrounding the location in which the initial single stranded polynucleotide molecule was immobilized.

Once more copies of the single stranded polynucleotide molecule and its complement have been synthesized by carrying out further rounds of amplification, i.e. further rounds of hybridization, extension and denaturation, then the boundary of the nucleic acid colony or cluster being generated will be able to be extended further, although the boundary of the colony formed is still limited to a relatively local area around the location in which the initial single stranded polynucleotide molecule was immobilized. For example the size of each amplified cluster may be 0.5-5 microns.

It can thus be seen that the method of the present invention allows the generation of a plurality of nucleic acid colonies from multiple single immobilized single stranded polynucleotide molecules and that the density of these colonies can be controlled by altering the proportions of modified capture/amplification oligonucleotides used to graft the surface of the solid support.

In one embodiment, the hybridization, extension and denaturation steps are all carried out at the same, substantially isothermal temperature. For example the temperature is from 37°C to about 75°C, particularly from 50°C to 70°C, yet more particularly from 60°C to 65°C. In a particular embodiment the substantially isothermal temperature may be the optimal temperature for the desired polymerase.

In a particular aspect, the method according to the first aspect of the invention is used to prepare clustered arrays of nucleic acid colonies, analogous to those described in U.S. Pat. No. 7,115,400, US 2005/0100900 A1, WO 00/18957 and WO 98/44151 by solid-phase amplification.

In yet another aspect more than one capture sequences and more than two amplification sequences, for example, at least three or four or more, different amplification primer sequences may be grafted to the solid support. In this manner more than one library, with common sequences which differ between the libraries, could be utilized to prepare clusters, such as, for example libraries prepared from two different patients. Whilst the cluster may overlap in space, they would be able to be sequenced one after the other due to the differences between the ends of the templates. For example, two different samples can be captured using two different capture sequences. These can be amplified from the same two amplification primers. The samples can be differentiated due to the two different capture sequences, which can be used as the sites for hybridization of two different sequencing primers. The use of different capture sequences thereby gives rise to a method of sample indexing using different sequencing primers.

Clustered arrays formed by the methods of the invention are suitable for use in applications usually carried out on ordered arrays such as micro-arrays. Such applications by way of non-limiting example include hybridization analysis, gene expression analysis, protein binding analysis, sequencing, genotyping, nucleic acid methylation analysis and the like. The clustered array may be sequenced before being used for downstream applications such as, for example, hybridization with fluorescent RNA or binding studies using fluorescent labelled proteins.

Sequencing Methods

The invention also encompasses methods of sequencing amplified nucleic acids generated by solid-phase amplification. Thus, the invention provides a method of nucleic acid sequencing comprising amplifying a pool of nucleic acid templates using solid-phase amplification as described above and carrying out a nucleic acid sequencing reaction to determine the sequence of the whole or a part of at least one amplified nucleic acid strand produced in the solid-phase amplification reaction.

Sequencing can be carried out using any suitable sequencing technique. A particularly useful method is one wherein nucleotides are added successively to a free 3' hydroxyl group, resulting in synthesis of a polynucleotide chain in the 5' to 3' direction. The nature of the nucleotide added may be determined after each nucleotide addition or at the end of the sequencing process. Sequencing techniques using sequencing by ligation, wherein not every contiguous base is sequenced, and techniques such as massively parallel signature sequencing (MPSS) where bases are removed from, rather than added to the strands on the surface are also within the scope of the invention.

The initiation point for the sequencing reaction may be provided by annealing of a sequencing primer to a product of the solid-phase amplification reaction. In this connection, one or both of the adaptors added during formation of the template library may include a nucleotide sequence which permits annealing of a sequencing primer to amplified products derived by whole genome or solid-phase amplification of the template library.

The products of solid-phase amplification reactions wherein both forward and reverse amplification primers are covalently immobilized on the solid surface are so-called bridged structures formed by annealing of pairs of immobilized polynucleotide strands and immobilized complementary strands, both strands being attached to the solid support at the 5' end. Arrays comprised of such bridged structures provide inefficient templates for typical nucleic acid sequencing techniques, since hybridization of a conventional sequencing primer to one of the immobilized strands is not favored compared to annealing of this strand to its immobilized complementary strand under standard conditions for hybridization.

In order to provide more suitable templates for nucleic acid sequencing, it may be advantageous to remove or displace substantially all or at least a portion of one of the immobilized strands in the bridged structure in order to generate a template which is at least partially single-stranded. The portion of the template which is single-stranded will thus be available for hybridization to a sequencing primer. The process of removing all or a portion of one immobilized strand in a 'bridged' double-stranded nucleic acid structure may be referred to herein as linearization, and is described in further detail in WO07010251, the contents of which are incorporated herein by reference in their entirety.

Bridged template structures may be linearized by cleavage of one or both strands with a restriction endonuclease or by cleavage of one strand with a nicking endonuclease. Other methods of cleavage can be used as an alternative to restriction enzymes or nicking enzymes, including inter alia chemical cleavage (e.g. cleavage of a diol linkage with periodate), cleavage of abasic sites by cleavage with endonuclease (for example 'USER', as supplied by NEB, part number M5505S), or by exposure to heat or alkali, cleavage of ribonucleotides incorporated into amplification products otherwise comprised of deoxyribonucleotides, photochemical cleavage or cleavage of a peptide linker.

Following the cleavage step, regardless of the method used for cleavage, the product of the cleavage reaction may be subjected to denaturing conditions in order to remove the portion(s) of the cleaved strand(s) that are not attached to the solid support. Suitable denaturing conditions, for example sodium hydroxide solution, formamide solution or heat, will be apparent to the skilled reader with reference to standard molecular biology protocols (Sambrook et al., supra; Ausubel et al. supra). Denaturation results in the production of a sequencing template which is partially or substantially single-stranded. A sequencing reaction may then be initiated by hybridization of a sequencing primer to the single-stranded portion of the template.

Thus, the invention encompasses methods wherein the nucleic acid sequencing reaction comprises hybridizing a sequencing primer to a single-stranded region of a linearized amplification product, sequentially incorporating one or more nucleotides into a polynucleotide strand complementary to the region of amplified template strand to be sequenced, identifying the base present in one or more of the incorporated nucleotide(s) and thereby determining the sequence of a region of the template strand.

One sequencing method which can be used in accordance with the invention relies on the use of modified nucleotides having removable 3' blocks, for example as described in WO04018497, US 2007/0166705A1 and U.S. Pat. No. 7,057,026. Once the modified nucleotide has been incorporated into the growing polynucleotide chain complementary to the region of the template being sequenced there is no free 3'-OH group available to direct further sequence extension and therefore the polymerase can not add further nucleotides. Once the nature of the base incorporated into the growing chain has been determined, the 3' block may be removed to allow addition of the next successive nucleotide. By ordering the products derived using these modified nucleotides, it is possible to deduce the DNA sequence of the DNA template. Such reactions can be done in a single experiment if each of the modified nucleotides has a different label attached thereto, known to correspond to the particular base, to facilitate discrimination between the bases added during each incorporation step. Alternatively, a separate reaction may be carried out containing each of the modified nucleotides separately.

The modified nucleotides may carry a label to facilitate their detection. A fluorescent label, for example, may be used for detection of modified nucleotides. Each nucleotide type may thus carry a different fluorescent label, for example, as described in WO07135368, the contents of which are incorporated herein by reference in their entirety. The detectable label need not, however, be a fluorescent label. Any label can be used which allows the detection of an incorporated nucleotide.

One method for detecting fluorescently labeled nucleotides comprises using laser light of a wavelength specific for the labelled nucleotides, or the use of other suitable sources of illumination. The fluorescence from the label on the nucleotide may be detected by a CCD camera or other suitable detection means. Suitable instrumentation for recording images of clustered arrays is described in WO07123744.

## Claims

1. A method for assessing cross-contamination in a multiplex sequencing reaction, the method comprising:

   obtaining a solution comprising a first template nucleic acid and obtaining a solution comprising a second template nucleic acid;
   contacting a first identifier nucleic acid to the first template nucleic acid and a second identifier nucleic acid to the second template nucleic acid;
   incorporating a first barcode oligonucleotide into both the first nucleic acid template and the first identifier nucleic acid;
   incorporating a second barcode oligonucleotide into both the second template nucleic acid and the second identifier nucleic acid;
   sequencing the template nucleic acids and the identifier nucleic acids; and
   confirming a correct association between the identifier nucleic acids and the barcode oligonucleotides.

2. The method according to claim 1, wherein prior to sequencing, the templates and the identifiers are attached to a substrate.

3. The method according to claim 2, wherein the templates and the identifiers are directly attached to the substrate.

4. The method according to claim 2, wherein the templates and the identifiers are indirectly attached to the substrate.

5. The method according to claim 1, further comprising, amplifying the templates and the identifiers.

6. The method according to claim 1, wherein sequencing is sequencing by synthesis.

7. The method according to claim 6, wherein the sequencing by synthesis is single molecule sequencing by synthesis.

8. The method according to claim 1, wherein sequencing comprises:

   hybridizing a primer to the templates and the identifiers to form template/primer duplexes and identifier/primer duplexes;
   contacting the duplexes with a polymerase enzyme in the presence of at least one detectably labeled nucleotide under conditions that permit the polymerase to add nucleotides to the primers of the duplexes in a template-dependent manner;
   detecting a signal from the incorporated labeled nucleotide; and
   sequentially repeating the contacting and detecting steps at least once, wherein sequential detection of incorporated labeled nucleotide determines the sequences of the template nucleic acids and the identifier nucleic acids.

9. The method according to claim 8, wherein the detectably labeled nucleotide is an optically labeled nucleotide.

10. The method according to claim 9, wherein the optically labeled nucleotide is a fluorescently labeled nucleotide.

11. The method according to claim 1, further comprising quantifying a contamination rate in the sequencing reaction by:

   determining a number of barcode reads in the sequencing reaction that are correctly correlated with the identifier nucleic acids, wherein the identifier nucleic acids are associated with the template nucleic acids, and the barcode reads are generated by a sequencing apparatus;
   determining a number of barcode reads in the sequencing reaction that are incorrectly correlated with the identifier nucleic acids; and
   obtaining a fraction of the two numbers, thereby quantifying a contamination rate in the sequencing reaction.

12. The method according to claim 1, wherein incorporating comprises a PCR reaction.

13. The method according to claim 1, wherein incorporating comprises a ligation reaction.

**Patentansprüche**

1. Verfahren zum Abschätzen von Querkontamination in einer Multiplex-Sequenzierungsreaktion, wobei das Verfahren umfasst:

   Erhalten einer Lösung, die eine erste Template-Nukleinsäure umfasst, und Erhalten einer Lösung, die eine zweite Template-Nukleinsäure umfasst;
   Kontaktieren einer ersten Identifizierungs-Nukleinsäure mit der ersten Template-Nukleinsäure und einer zweiten Identifizierungs-Nukleinsäure mit der zweiten Template-Nukleinsäure;
   Einbauen eines ersten Barcode-Oligonukleotids in sowohl die erste Template-Nukleinsäure als auch die erste Identifizierungs-Nukleinsäure;
   Einbauen eines zweiten Barcode-Oligonukleotids in sowohl die zweite Template-Nukleinsäure als auch die zweite Identifizierungs-Nukleinsäure;
   Sequenzieren der Template-Nukleinsäuren und der Identifizierungs-Nukleinsäuren; und
   Bestätigen einer korrekten Verbindung zwischen den Identifizierungs-Nukleinsäuren und den Barcode-Oligonukleotiden.

2. Verfahren nach Anspruch 1, wobei die Templates und die Identifizierer vor einem Sequenzieren an einem Substrat befestigt werden.

3. Verfahren nach Anspruch 2, wobei die Templates und die Identifizierer direkt an dem Substrat befestigt werden.

4. Verfahren nach Anspruch 2, wobei die Templates und die Identifizierer indirekt an dem Substrat befestigt werden.

5. Verfahren nach Anspruch 1, weiter umfassend Amplifizieren der Templates und der Identifizierer.

6. Verfahren nach Anspruch 1, wobei Sequenzieren Sequenzieren durch Synthese ist.

7. Verfahren nach Anspruch 6, wobei das Sequenzieren durch Synthese ein Einzelmolekülsequenzieren durch Synthese ist.

**8.** Verfahren nach Anspruch 1, wobei Sequenzieren umfasst:

Hybridisieren eines Primers an die Templates und die Identifizierer, um Template/Primer-Duplexe und Identifizierer/Primer-Duplexe zu bilden;
Kontaktieren der Duplexe mit einem Polymerase-Enzym in der Anwesenheit von wenigstens einem detektierbar markierten Nukleotid unter Bedingungen, die der Polymerase erlauben, Nukleotide zu den Primern der Duplexe in einer Template-abhängigen Weise hinzuzufügen;
Detektieren eines Signals von dem eingebauten markierten Nukleotid; und
sequenziell wenigstens einmal Wiederholen der Kontaktierungs- und Detektierungsschritte, wobei eine sequenzielle Detektierung von eingebauten markierten Nukleotiden die Sequenzen der Template-Nukleinsäuren und der Identifizierungs-Nukleinsäuren bestimmt.

**9.** Verfahren nach Anspruch 8, wobei das detektierbar markierte Nukleotid ein optisch markiertes Nukleotid ist.

**10.** Verfahren nach Anspruch 9, wobei das optisch markierte Nukleotid ein fluoreszierend markiertes Nukleotid ist.

**11.** Verfahren nach Anspruch 1, weiter umfassend Quantifizieren einer Kontaminationsrate in der Sequenzierungsreaktion durch:

Bestimmen einer Zahl von Barcode-Lesungen in der Sequenzierungsreaktion, die mit den Identifizierungs-Nukleinsäuren korrekt korreliert sind, wobei die Identifizierungs-Nukleinsäuren mit den Template-Nukleinsäuren verbunden sind, und die Barcode-Lesungen durch eine Sequenzierungsvorrichtung generiert werden;
Bestimmen einer Zahl von Barcode-Lesungen in der Sequenzierungsreaktion, die mit den Identifizierungs-Nukleinsäuren inkorrekt korreliert sind; und
Erhalten eines Bruchs der zwei Zahlen, wodurch eine Kontaminationsrate in der Sequenzierungsreaktion quantifiziert wird.

**12.** Verfahren nach Anspruch 1, wobei das Einbauen eine PCR-Reaktion umfasst.

**13.** Verfahren nach Anspruch 1, wobei das Einbauen eine Ligationsreaktion umfasst.

**Revendications**

**1.** Procédé d'évaluation d'une contamination croisée dans une réaction de séquençage multiplex, le procédé comprenant :

l'obtention d'une solution comprenant un premier acide nucléique matrice et l'obtention d'une solution comprenant un second acide nucléique matrice ;
la mise en contact d'un premier acide nucléique identifiant avec le premier acide nucléique matrice et d'un second acide nucléique identifiant avec le second acide nucléique matrice ;
l'incorporation d'un premier oligonucléotide à code à barres à la fois au premier acide nucléique matrice et au premier acide nucléique identifiant ;
l'incorporation d'un second oligonucléotide à code à barres à la fois au second acide nucléique matrice et au second acide nucléique identifiant ;
le séquençage des acides nucléiques matrices et des acides nucléiques identifiants ; et
la confirmation d'une association correcte entre les acides nucléiques identifiants et les oligonucléotides à code à barres.

**2.** Procédé selon la revendication 1, dans lequel, avant le séquençage, les matrices et les identifiants sont fixés à un substrat.

**3.** Procédé selon la revendication 2, dans lequel les matrices et les identifiants sont directement fixés au substrat.

**4.** Procédé selon la revendication 2, dans lequel les matrices et les identifiants sont indirectement fixés au substrat.

**5.** Procédé selon la revendication 1, comprenant en outre l'amplification des matrices et des identifiants.

**6.** Procédé selon la revendication 1, dans lequel le séquençage est un séquençage par synthèse.

**7.** Procédé selon la revendication 6, dans lequel le séquençage par synthèse est un séquençage de molécules individuelles par synthèse.

**8.** Procédé selon la revendication 1, dans lequel le séquençage comprend :

l'hybridation d'une amorce avec les matrices et les identifiants pour former des duplex matrice/amorce et des duplex identifiant/amorce ;
la mise en contact des duplex avec un enzyme de polymérase en présence d'au moins un nucléotide marqué de manière détectable dans des conditions qui permettent à la polymérase d'ajouter des nucléotides aux amorces des duplex de manière dépendant de la matrice ; la détection d'un signal provenant du nucléotide marqué incorporé ; et la répétition séquentielle des étapes de mise en contact et de détection au moins une fois, dans lequel la détection séquentielle du nucléotide marqué incorporé détermine les séquences des acides nucléiques matrices et des acides nucléiques identifiants.

9. Procédé selon la revendication 8, dans lequel le nucléotide marqué de manière détectable est un nucléotide marqué en mode optique.

10. Procédé selon la revendication 9, dans lequel le nucléotide marqué en mode optique est un nucléotide à marquage fluorescent.

11. Procédé selon la revendication 1, comprenant en outre la quantification d'un taux de contamination dans la réaction de séquençage par :

   détermination d'un certain nombre de lectures de code à barres dans la réaction de séquençage qui sont en corrélation correcte avec les acides nucléiques identifiants, dans lequel les acides nucléiques identifiants sont associés aux acides nucléiques matrices et les lectures de code à barres sont générées par un appareil de séquençage ; détermination d'un certain nombre de lectures de code à barres dans la réaction de séquençage qui sont en corrélation incorrecte avec les acides nucléiques identifiants ; et obtention d'une fraction des deux nombres, en quantifiant de la sorte un taux de contamination dans la réaction de séquençage.

12. Procédé selon la revendication 1, dans lequel l'incorporation comprend une réaction PCR.

13. Procédé selon la revendication 1, dans lequel l'incorporation comprend une réaction de ligature.

Patient A   Patient B   Patient A   Patient B

DNA Isolation

Shearing

Barcoding

Pooling and simultaneous sequencing

Genomic Read(s)

Barcode Read

PRIOR ART

# FIG. 1

EP 2 691 546 B1

PRIOR ART

FIG. 2

17

Patient A      Patient B

Sample ID's

Contamination
Identifiable by unexpected
Barcode-ID pair

FIG. 3

Primer Stock #1
Identifier #1

Primer Stock #2
Identifier #2

PCR

Sequencing
(Counting)

**Sequence**          **Count**

200        ⇒  Stock #1 contaminated

100

500    ⇒  Stock #1 not contaminated

# FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 07716911 A
- US 20020190663 A1
- WO 2004083819 A2
- WO 9619586 A
- US 6235475 B, Brenner
- US 6138077 A
- US 6352828 B
- US 5636400 A
- US 6172214 B
- US 7393665 B
- US 7544473 B
- US 5846719 A
- US 5695934 A
- US 5604097 A
- US 6150516 A
- US RE39793 E
- US 7537897 B
- US 6172218 B
- US 5863722 A
- US 20090226975 A, Sabot
- US 7115400 B, Adessi
- US 20050100900 A, Kawashima
- US 20070128624 A
- US 20050100900 A1
- WO 0018957 A
- WO 9844151 A
- WO 07010251 A
- WO 04018497 A
- US 20070166705 A1
- US 7057026 B
- WO 07135368 A
- WO 07123744 A

### Non-patent literature cited in the description

- **MANIATIS et al.** Molecular Cloning: A Laboratory Manual. Cold Spring Harbor, 1982, 280-281
- **SAMBROOK ; RUSSELL.** Molecular Cloning, A Laboratory Manual. Cold Spring Harbor Laboratory Press, 2001
- **AUSUBEL et al.** Current Protocols in Molecular Biology. John Wiley and Sons, 1998